# EUROPEAN PATENT SPECIFICATION

(11) **EP 2 015 748 B1**
(45) Date of publication and mention of the grant of the patent: **04.07.2012**
(21) Application number: 07760927.9
(22) Date of filing: 19.04.2007
(51) Int. Cl.: A61K 31/4439, A61K 31/454, A61K 31/496, A61K 31/5377, A61P 35/00

(54) **A C-Kit kinase inhibitor for use in the treatment of gastrointestinal stromal tumor or mastocytosis**
Ein C-Kit-Kinase Inhibitor zur Verwendung in der Behandlung von gastrointestinalem Stromatumor oder Mastozytose
Un inhibiteur de la kinase C-kit pour son utilisation dans le traitement des tumeurs stromales gastro-intestinales ou de la mastocytose

(30) Priority: 20.04.2006 US 793471 P
(43) Date of publication of application: 21.01.2009
(60) Divisional application: 11161399.8
(73) Proprietor: Janssen Pharmaceutica NV, 2340 Beerse (BE)
(72) Inventor: ILLIG, Carl, R., Phoenixville, PA 19460 (US); BALLENTINE, Shelley, K., Lansdale, PA 19446 (US); CHEN, Jingsheng, Exton, PA 19341 (US); MEEGALLA, Sanath, K., Boothwyn, PA 19061 (US); WALL, Mark, J, Harleysville, PA 19438 (US); WILSON, Kenneth, J., Media, PA 19063 (US); RUDOLPH, M., Jonathan, Doylestown, PA 18901-6232 (US); DESJARLAIS, Renee, L., Saint Davies, 19087 (US); MOLLOY, Christopher, J., Yardley, PA 19067 (US); MANTHEY, Carl, L., Downington, PA 19335 (US); FLORES, Christopher, Lansdale, PA 19446 (US)
(74) Representative: Warner, James Alexander
(86) International application number: PCT/US2007/066985
(87) International publication number: WO 2007/124369

(56) References cited:
- WO-A-2006/047277
- WO-A-2006/138155
- WO-A-2007/048088

## Description

### FIELD OF THE INVENTION

The present invention relates to compounds for use in methods of treating in a subject gastrointestinal stromal tumor or mastocytosis

### BACKGROUND OF THE INVENTION

Protein kinases are enzymatic components of the signal transduction pathways which catalyze the transfer of the terminal phosphate from ATP to the hydroxy group of tyrosine, serine and/or threonine residues of proteins. Thus, compounds which inhibit protein kinase functions are valuable tools for assessing the physiological consequences of protein kinase activation. The overexpression or inappropriate expression of normal or mutant protein kinases in mammals has been a topic of extensive study and has been demonstrated to play a significant role in the development of many diseases, including diabetes, angiogenesis, psoriasis, restenosis, ocular diseases, schizophrenia, rheumatoid arthritis, atherosclerosis, cardiovascular disease and cancer. The cardiotonic benefits of kinase inhibition has also been studied. In sum, inhibitors of protein kinases have particular utility in the treatment of human and animal disease.

The receptor tyrosine kinase C-KIT and its ligand Stem Cell Factor (SCF) are essential for hemoatpoiesis, melanogenesis and fertility. SCF acts at multiple levels of the hemoatpoietic hierarchy to promote cell survival, proliferation, differentiation, adhesion and functional activation. It is of particular importance in the mast cell and erythroid lineages, but also acts on multipotential stem and progenitor cells, mcgakaryocytes, and a subset of lymphoid progenitors (see, Int J Biochem Cell Biol. 1999 Oct;31(10):1037-51). Sporadic mutations of C-KIT as well as autocrine/paracrine activation mechanisms of the SCF/C-KIT pathway have been implicated in a variety of malignancies. Activation of C-KIT contributes to metastases by enhancing tumor growth and reducing apoptosis. Additionally, C-KIT is frequently mutated and activated in gastrointestinal stromal tumors (GISTs), and ligand-mediated activation of C-KIT is present in some lung cancers (see, Leuk Res. 2004 May;28 Suppl 1:S11-20). The C-KIT receptor also is expressed on more than 10% of blasts in 64% of de novo acute myelogenous leukemias (AMLs) and 95% of relapsed AMLs. C-kit mediates proliferation and anti-apoptotic effects in AML (see, Curr Hematol Rep. 2005 Jan;4(1):51-8).

C-Kit expression has been documented in a wide variety of human malignancies, including mastocytosis, mast cell leukemia, gastrointestinal stromal tumour, sinonasal natural killer/T-cell lymphoma, seminoma, dysgerminoma, thyroid carcinoma; small-cell lung carcinoma, malignant melanoma, adenoid cystic carcinoma, ovarian carcinoma, acute myelogenous leukemia, anaplastic large cell lymphoma, angiosarcoma, endometrial carcinoma, pediatric T-cell ALL, lymphoma, breast carcinoma and prostate carcinoma. See, Heinrich, Michael C. et al. Review Article: Inhibition of KIT Tyrosine Kinase Activity: A Novel Molecular Approach to the Treatment of KIT-Positive Malignancies. Jouranl of Clinical Oncology, Vol 20, No 6 (March 15),2002: pp 1692-1703.

### SUMMARY OF THE INVENTION

The present invention provides compounds for use in methods of treating in a subject gastrointestinal stromal tumor or mastocytosis.

Other features and advantages of the invention will be apparent from the following detailed description of the invention and from the claims.

### DETAILED DESCRIPTION OF THE INVENTION

The terms "comprising", "including", and "containing" are used herein in their open, non-limited sense.

### ABBREVIATIONS

As used herein, the following abbreviations are intended to have the following meanings (additional abbreviations are provided where needed throughout the Specification):
- ATP: adenosine triphosphate
- Boc or BOC: *tert*-butoxycarbonyl
- DCM: dichloromethane
- DMF: dimethylformamide
- DMSO: dimethylsulfoxide
- DIEA: diisopropylethylamine
- EDCI: 1-(3-dimethylaminopropyl)-3-ethylcarbodiimide hydrochloride
- EDTA: ethylenediaminetetraaceticacid
- EtOAc: ethyl acetate
- FP: fluorescence polarization
- HOBT or HOBt: 1-hydroxybenzotriazole hydrate
- LC/MS (ESI): Liquid chromatography/mass spectrum (electrospray ionization)
- MeOH: Methyl alcohol
- NMR: nuclear magnetic resonance
- RT: room temperature
- TFA: trifluoroacetic acid
- THF: tetrahydrofuran
- TLC: thin layer chromatography

### DEFINITIONS

The term "**alkyl**" refers to both linear and branched chain radicals of up to 12 carbon atoms, preferably up to 6 carbon atoms, unless otherwise indicated, and includes, but is not limited to, methyl, ethyl, propyl, isopropyl, butyl, isobutyl, sec-butyl, tert-butyl, pentyl, isopentyl, hexyl, isohexyl, heptyl, octyl, 2,2,4-trimethylpentyl, nonyl, decyl, undecyl and dodecyl.

The term "**hydroxyalkyl**" refers to both linear and branched chain radicals of up to 6 carbon atoms, in which one hydrogen atom has been replaced with an OH group.

The term "**hydroxyalkylamino**" refers to an hydroxyalkyl group in which one hydrogen atom from the carbon chain has been replaced with an amino group, wherein the nitrogen is the point of attachment to the rest of the molecule.

The term "**cycloalkyl**" refers to a saturated or partially unsaturated ring composed of from 3 to 8 carbon atoms. Up to four alkyl substituents may optionally be present on the ring. Examples include cyclopropyl, 1,1-dimethyl cyclobutyl, 1,2,3-trimethylcyclopentyl, cyclohexyl, cyclopentenyl, cyclohexenyl, and 4,4-dimethyl cyclohexenyl.

The term "**dihydrosulfonopyranyl**" refers to the following radical:

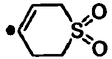

The term "**hydroxyalkyl**" refers to at least one hydroxyl group bonded to any carbon atom along an alkyl chain.

The term "**aminoalkyl**" refers to at least one primary or secondary amino group bonded to any carbon atom along an alkyl chain, wherein an alkyl group is the point of attachment to the rest of the molecule.

The term **"alkylamino"** refers to an amino with one alkyl substituent, wherein the amino group is the point of attachment to the rest of the molecule.

The term "**dialkylamino**" refers to an amino with two alkyl substituents, wherein the amino group is the point of attachment to the rest of the molecule.

The term "**heteroaromatic**" or "**heteroaryl**" refers to 5- to 7-membered mono- or 8-to 10-membered bicyclic aromatic ring systems, any ring of which may consist of from one to four heteroatoms selected from N, O or S where the nitrogen and sulfur atoms can exist in any allowed oxidation state. Examples include benzimidazolyl, benzothiazolyl, benzothienyl, benzoxazolyl, furyl, imidazolyl, isothiazolyl, isoxazolyl, oxazolyl, pyrazinyl, pyrazolyl, pyridyl, pyrimidinyl, pyrrolyl, quinolinyl, thiazolyl and thienyl.

The term **"heteroatom"** refers to a nitrogen atom, an oxygen atom or a sulfur atom wherein the nitrogen and sulfur atoms can exist in any allowed oxidation states.

The term "**alkoxy**" refers to straight or branched chain radicals of up to 12 carbon atoms, unless otherwise indicated, bonded to an oxygen atom. Examples include methoxy, ethoxy, propoxy, isopropoxy and butoxy.

The term "**aryl**" refers to monocyclic or bicyclic aromatic ring systems containing from 6 to 12 carbons in the ring. Alkyl substituents may optionally be present on the ring. Examples include benzene, biphenyl and napththalene.

The term "**aralkyl**" refers to a C₁₋₆ alkyl group containing an aryl substituent. Examples include benzyl, phenylethyl or 2-naphthylmethyl.

The term "**sulfonyl**" refers to the group ―S(O)₂Rₐ, where Rₐ is hydrogen, alkyl, cycloalkyl, haloalkyl, aryl, aralkyl, heteroaryl and heteroaralkyl. A "sulfonylating agent" adds the ―S(O)₂Rₐ group to a molecule.

Processes for preparing compounds of Formula 1 are disclosed : 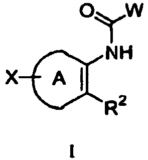 or a solvate, hydrate, tautomer or pharmaceutically acceptable salt thereof, wherein:
**A** is
   phenyl or pyridyl, either of which may be substituted with one of chloro, fluoro, methyl, -N₃, -NH₂, -NH(alkyl), -N(alkyl)₂, -S(alkyl), -O(alkyl), or 4-aminophenyl;
**W** is
   pyrrolyl (including 1*H-*pyrrol-2-yl), imidazolyl, (including 1*H*-imidazol-2-yl), isoxazolyl, oxazolyl, 1,2,4 triazolyl, or furanyl (including furan-2-yl), any of which may be connected through any carbon atom, wherein the pyrrolyl, imidazolyl, isoxazolyl, oxazolyl, 1,2,4 triazolyl, or furanyl may contain one - Cl, ―CN, -NO₂, -OMe, or -CF₃ substitution, connected to any other carbon;
**R²** is
   cycloalkyl (including cyclohexenyl, cyclopentenyl), thiophenyl, dihydrosulfonopyranyl, phenyl, furanyl, tetrahydropyridyl, or dihydropyranyl, any of which may be independently substituted with one or two of each of the following: chloro, fluoro, and C₍₁₋₃₎alkyl (including 4,4-dimethyl cyclohexenyl, 4-methyl cyclohexenyl, 2-methyl thiophenyl, 3-methyl thiophenyl), with the proviso that tetrahydropyridyl is connected to the ring A through a carbon-carbon bond;
**X** is 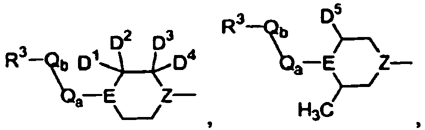 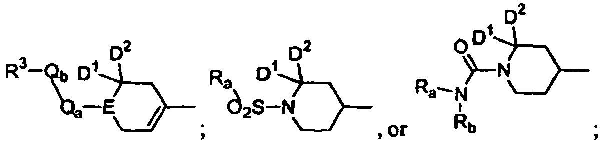
**Z** is
   CH or N;
**D¹** and **D²** are
   each hydrogen or taken together form a double bond to an oxygen;
**D³** and **D⁴** are
   each hydrogen or taken together form a double bond to an oxygen;
**D⁵** is
   hydrogen or -CH₃, wherein said ―CH₃ may be relatively oriented *syn* or *anti*;
**Rₐ** and **R_{b}** are independently
   hydrogen, cycloalkyl, haloalkyl, aryl, aralkyl, heteroaryl, or heteroaralkyl;
**E** is
   N, S, O, SO or SO₂, with the proviso that E may not be N if the following three conditions are simultaneously met: Qₐ is absent, Q_{b} is absent, and **R³** is an amino group or cyclic amino radical wherein the point of attachment to E is N;
**Qₐ** is
   absent, -CH₂-, -CH₂CH₂-, or C(O);
**Q_{b}** is
   absent, -NH-, -CH₂-, -CH₂CH₂-, or C(O), with the proviso that Q_{b} may not be C(O) if Qₐ is C(O), and further provided that Q_{b} may not be ― NH- if E is N and Qₐ is absent, further provided that Q_{b} may not be - NH- if R³ is an amino group or cyclic amino radical wherein the point of attachment to Q_{b} is N;
**R³** is
   hydrogen, phenyl, hydroxyalkylamino (including 2-hydroxy ethylamino), (hydroxyalkyl)₂amino, hydroxyalkyl(alkyl)amino (including 1-hydroxyeth-2-yl(methyl)amino), alkylamino (including methylamino), aminoalkyl (including 2-amino isopropyl), dihydroxyalkyl (including 1,3-dihydroxy isopropyl, 1,2-dihydroxy ethyl), alkoxy (including methoxy), dialkylamino (including dimethylamino), hydroxyalkyl (including 1-hydroxy eth-2-yl), - COOH, -CONH₂, -CN, -SO₂-alkyl-R⁴ (including -SO₂CH₃), -NH₂, or a 5 or six membered ring which contains at least one heteroatom N and may optionally contain an additional heteromoiety selected from *S*, *SO₂, N,* and *O*, and the 5 or 6 membered ring may be saturated, partially unsaturated or aromatic (including piperidinyl, morpholinyl, imidazolyl, and pyridyl) wherein aromatic nitrogen in the 5 or 6 membered ring may be present as N-oxide (including pyridyl N-oxide), and the 5 or 6 membered ring may be optionally substituted with methyl, halogen, alkylamino, or alkoxy (including 1 methyl imidazolyl); R³ may also be absent, with the proviso that R³ is not absent when E is nitrogen;
**R⁴** is
   hydrogen, -OH, alkoxy, carboxy, carboxamido, or carbamoyl.

The compounds of the present invention are :
4-cyano-1H-imidazole-2-carboxylic acid {2-cyclohex-1-enyl-4-[1-(2-dimethylaminoacetyl)-piperidin-4-yl]-phenyl}-amide,
and solvates, hydrates, tautomers and pharmaceutically acceptable salts thereof.

As used herein, the term "the compounds of the present invention" shall also include solvates, hydrates, tautomers or pharmaceutically acceptable salts thereof.

### PHARMACEUTICALLY ACCEPTABLE SALTS

As stated, the compounds of the present invention may also be present in the form of pharmaceutically acceptable salts.

For use in medicines, the salts of the compounds of the present invention refer to non-toxic "pharmaceutically acceptable salts." FDA approved pharmaceutically acceptable salt forms (*Ref.* International J. Pharm. 1986, 33, 201-217; J. Pharm. Sci., 1977, Jan, 66(1), p1) include pharmaceutically acceptable acidic/anionic or basic/cationic salts.

Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate and triethiodide. Organic or inorganic acids also include, and are not limited to, hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic or trifluoroacetic acid.

Pharmaceutically acceptable basic/cationic salts include, and are not limited to aluminum, 2-amino-2-hydroxymethyl-propane-1,3-diol (also known as tris(hydroxymethyl)aminomethane, tromethane or "TRIS"), ammonia, benzathine, *t*-butylamine, calcium, calcium gluconate, calcium hydroxide, chloroprocaine, choline, choline bicarbonate, choline chloride, cyclohexylamine, diethanolamine, ethylenediamine, lithium, LiOMe, L-lysine, magnesium, meglumine, NH₃, NH₄OH, N-methyl-D-glucamine, piperidine, potassium, potassium-*t*-butoxide, potassium hydroxide (aqueous), procaine, quinine, sodium, sodium carbonate, sodium-2-ethylhexanoate (SEH), sodium hydroxide,or zinc.

### PRODRUGS

Prodrugs of the compounds of the present invention are also disclosed. In general, such prodrugs will be functional derivatives of the compounds which are readily convertible *in vivo* into an active compound. Thus, in the methods of treatment for which the compounds of the present invention are used, the term "administering" shall encompass the means for treating, ameliorating or preventing a syndrome, disorder or disease described herein with the compounds of the present invention or a prodrug thereof Conventional procedures for the selection and preparation of suitable prodrug derivatives are described in, for example, "Design of Prodrugs", ed. H. Bundgaard, Elsevier, 1985.

### STEREOCHEMICAL ISOMERS

One skilled in the art will recognize that some compounds of the present invention have one or more asymmetric carbon atoms in their structure. It is intended that the present invention include within its scope single enantiomer forms of the compounds of the present invention, racemic mixtures, and mixtures of enantiomers in which an enantiomeric excess is present.

The term "single enantiomer" as used herein defines all the possible homochiral forms which the compounds of the present invention and their N-oxides, addition salts, quaternary amines, and physiologically functional derivatives may possess.

Stereochemically pure isomeric forms may be obtained by the application of art known principles. Diastereoisomers may be separated by physical separation methods such as fractional crystallization and chromatographic techniques, and enantiomers may be separated from each other by the selective crystallization of the diastereomeric salts with optically active acids or bases or by chiral chromatography. Pure stereoisomers may also be prepared synthetically from appropriate stereochemically pure starting materials, or by using stereoselective reactions.

The term "isomer" refers to compounds that have the same composition and molecular weight but differ in physical and/or chemical properties. Such substances have the same number and kind of atoms but differ in structure. The structural difference may be in constitution (geometric isomers) or in an ability to rotate the plane of polarized light (enantiomers).

The term "stereoisomer" refers to isomers of identical constitution that differ in the arrangement of their atoms in space. Enantiomers and diastereomers are examples of stereoisomers.

The term "chiral" refers to the structural characteristic of a molecule that makes it impossible to superimpose it on its mirror image.

The term "enantiomer" refers to one of a pair of molecular species that are mirror images of each other and are not superimposable.

The term "diastereomer" refers to stereoisomers that are not mirror images.

The symbols "R" and "*S*" represent the configuration of substituents around a chiral carbon atom(s).

The term "racemate" or "racemic mixture" refers to a composition composed of equimolar quantities of two enantiomeric species, wherein the composition is devoid of optical activity.

The term "homochiral" refers to a state of enantiomeric purity.

The term "optical activity" refers to the degree to which a homochiral molecule or nonracemic mixture of chiral molecules rotates a plane of polarized light.

It is to be understood that the various substituent stereoisomers, geometric isomers and mixtures thereof used to prepare the compounds of the present invention are either commercially available, can be prepared synthetically from commercially available starting materials or can be prepared as isomeric mixtures and then obtained as resolved isomers using techniques well-known to those of ordinary skill in the art.

The isomeric descriptors "R," and "S" are used as described herein for indicating atom configuration(s) relative to a core molecule and are intended to be used as defined in the literature (IUPAC Recommendations for Fundamental Stereochemistry (Section E), Pure Appl. Chem., 1976, 45:13-30).

The compounds of the present invention may be prepared as an individual isomer by either isomer-specific synthesis or resolved from an isomeric mixture. Conventional resolution techniques include forming the free base of each isomer of an isomeric pair using an optically active salt (followed by fractional crystallization and regeneration of the free base), forming an ester or amide of each of the isomers of an isomeric pair (followed by chromatographic separation and removal of the chiral auxiliary) or resolving an isomeric mixture of either a starting material or a final product using preparative TLC (thin layer chromatography) or a chiral HPLC column.

### POLYMORPHS AND SOLVATES

Furthermore, the compounds of the present invention may have one or more polymorph or amorphous crystalline forms. In addition, the compounds may form solvates, for example with water (i.e., hydrates) or common organic solvents. As used herein, the term "solvate" means a physical association of the compounds of the present invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. The term "solvate" is intended to encompass both solution-phase and isolatable solvates. Non-limiting examples of suitable solvates include ethanolates, methanolates, and the like.

It is intended that the present invention include within its scope solvates of the compounds of the present invention. Thus, in the methods of treatment for which the compounds of the present invention are used, the term "administering" shall encompass the compounds of the present invention or a solvate thereof for use in a method of treating, compounds of the present invention or a solvate thereof.

### N-OXIDES

The compounds of the present invention may be converted to the corresponding N-oxide form following art-known procedures for converting a trivalent nitrogen into its N-oxide form. Said N-oxidation reaction may generally be carried out by reacting the starting material with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *t*butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

### TAUTOMERIC FORMS

The compounds of the present invention may also exist in their tautomeric forms.

### PREPARATION OF THE COMPOUNDS OF THE PRESENT INVENTION

During any of the processes for preparation of the compounds of the present invention, it may be necessary and/or desirable to protect sensitive or reactive groups on any of the molecules concerned. This may be achieved by means of conventional protecting groups, such as those described in Protecting Groups, P. Kocienski, Thieme Medical Publishers, 2000; and T.W. Greene & P.G.M. Wuts, Protective Groups in Organic Synthesis, 3rd ed. Wiley Interscience, 1999. The protecting groups may be removed at a convenient subsequent stage using methods known in the art.

### Methods of Preparation

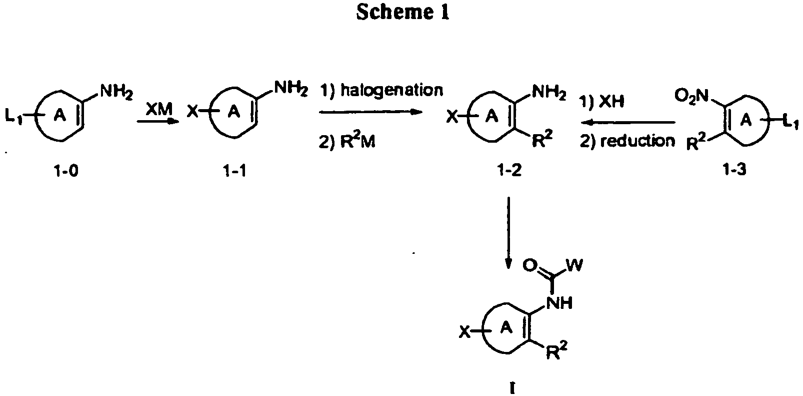

Scheme 1 illustrates general methodology for the preparation of compounds of Formula I. Compounds of Formula 1-2 can be obtained by ortho-halogenation, preferably bromination, of amino compounds of Formula 1-1 followed by metal-catalyzed coupling reactions with boronic acids or boronate esters (Suzuki reactions, where R²M is R²B(OH)₂ or a boronic ester) or tin reagents (Stille reactions, where R²M is R²Sn(alkyl)₃) (for reviews, see N. Miyaura, A. Suzuki, Chem. Rev., 95:2457 (1995), J. K. Stille, Angew. Chem, Int. Ed. Engl., 25: 508024 (1986) and A. Suzuki in Metal-Catalyzed Coupling Reactions, F. Deiderich, P. Stang, Eds., Wiley-VCH, Weinheim (1988)). Compounds of formula 1-1 may be commercially available, or the above palladium mediated cross-coupling reactions described above may be used to generate compounds of Formula 1-1 from starting material 1-0.

Preferred conditions for the bromination of 1-1 are N-bromosuccinimide (NBS) in a suitable solvent such as *N,N*-dimethylformamide (DMF), dichloromethane (DCM) or acetonitrile. Metal-catalyzed couplings, preferably Suzuki reactions, can be performed according to standard methodology, preferably in the presence of a palladium catalyst such as tetrakis(triphenylphosphine)palladium(0) (Pd(PPh₃)₄), an aqueous base such aq. Na₂CO₃, and a suitable solvent such as toluene, ethanol, dimethoxyethane (DME), or DMF.

Compounds of Formula I can be prepared by reaction of compounds of Formula 1-2 with carboxylic acids WCOOH according to standard procedures for amide bond formation (for a review, see: M. Bodansky and A. Bodansky, The Practice of Peptide Synthesis, Springer-Verlag, NY (1984)) or by reaction with acid chlorides WCOCI or activated esters WCO₂Rq (where Rq is a leaving group such as pentafluorophenyl or N-succinimide). The preferred reaction conditions for coupling with WCOOH are: when W is a furan, oxalyl chloride in DCM with DMF as a catalyst to form the acid chloride WCOCl and then coupling in the presence of a trialkylamine such as DIEA; when W is a pyrrole, 1-(3-dimethylaminopropyl)-3-ethylcarbodiimide hydrochloride (EDCI) and 1-hydroxybenzotriazole-6-sulfonamidomethyl hydrochloride (HOBt); and when W is an imidazole, the preferred conditions are bromotripyrrolidinophosphonium hexafluorophosphate (PyBrOP) and diisopropylethylamine (DIEA) in DCM.

It is understood that the optional substitution present on ring A in Formula I may be present in the starting materials 1-1 or 1-3 and, in such cases, would be carried through the synthesis outlined in Scheme 1. Alternatively various substituents on compounds of Formula I may be introduced in a number of ways described below to provide the optional substitution listed for Formula I. The leaving group "L₁" present on ring A in Formula 1-0 or 1-3, can be substituted before or at any step during Scheme 1. When such leaving groups (preferably fluoro or chloro) are activated by the nitro group of Formula 1-3 for nucleophilic attack, they can undergo direct nucleophilic aromatic substitution by ammonia and azide anion or by amines, alcohols, thiols and other nucleophiles in the presence of a suitable base such as K₂CO₃, *N,N*-diisopropylethylamine (DIEA) or NEt₃. When the leaving group is suitable for metal-catalyzed couplings (preferably bromo or trifluoromethane-sulfonyloxy), a number of cross-coupling reactions (such as Suzuki or Stille reactions as discussed above for the introduction of R²) may be performed. Other metal-catalyzed coupling reactions that can be employed include aromatic and heteroaromatic amination and amidation (for reviews, see: S. L. Buchwald, et al, Top. Curr. Chem., 219:131-209 (2001) and J. F. Hartwig in "Organopalladium Chemistry for Organic Synthesis," Wiley Interscience, NY (2002). Additional metal catalyzed cross coupling reactions with 2,4,6-trimethyl-cyclotriboroxane may be employed if L₁ is bromo, iodo, or chloro activated by nitro to generate optional methyl substitution (see M. Gray, et al, Tetrahedron Lett., 41: 6237-40 (2000)).

In some cases, the initial substituents can be further dcrivatizcd as described below to provide the final substitution of Formula I.

An alternative method for the introduction of nitrogen-containing heterocyclic substituents onto ring A is to form the heterocycle from an amino group on ring A. The amino group may be originally present in the starting material in a protected or unprotected form or may result from the reduction of a nitro group which also can be either originally present in the starting material or attached by a nitration reaction. In addition, the amino group may be formed by reduction of an azide group which can be present in the starting material or may result from nucleophilic aromatic substitution of an activated halide by azide anion as mentioned above. The amino group may also result from nucleophilic aromatic substitution of an activated halide (in, for example a nitrohalo compound) by ammonia or by the anion of a protected ammonia equivalent, for example, t-butyl carbamate. If introduced in protected form, the amine can be deprotected according to standard literature methods. (For examples of amine protecting groups and deprotection methods see: Theodora W. Greene and Peter G. M. Wuts, John Wiley and Sons, Inc., NY (1991).) The ring-forming reaction involves treatment of the aniline amino group with a suitable optionally substituted di-electrophile, preferably a dihalide or dicarbonyl compound, which results in two substitutions on the amino group to form an optionally substituted heterocycle. In the case of dihalides, any of a number of suitable bases can be added as an acid scavenger such as potassium carbonate, sodium hydroxide, or, a trialkylamine such as triethylamine. Thus, treatment with a bis(2-haloethyl)amine such as bis(2-chloroethyl)amine or bis(2-bromoethyl)amine would afford a piperazine ring (see, for example, J. Med. Chem., 29: 640-4 (1986) and J. Med. Chem., 46: 2837 (2003)). Optional substitution on the amine nitrogen of the reagent would incorporate optional substitution on the terminal amine of the piperazine. For example, treatment with *N,N*-bis(2-chloroethyl)aniline would give an *N*-phenylpiperazino group. Treatment with a bis(2-haloethyl)ether or bis(2-haloethyl)thioether would afford a morpholine or thiomorpholine ring, respectively.

Another alternative method to direct substitution to introduce heterocyclic substituents onto ring A is to form the heterocycle from an aldehyde (i.e. from a formyl group on ring A). The formyl group may be originally present in the starting material in a protected or unprotected form or may result from or any of a number of formylation reactions known in the literature including a Vilsmeier-Haack reaction (for a review of formylation chemistry, see: G. A. Olah, et al, Chem Rev., 87: (1987)) or by para-formylation of nitroaromatics (see: A. Katritsky and L. Xie, Tetrahedron Lett., 37:347-50 (1996)).

Finally it is understood that compounds of Formula 1 may be further derivatized. Protecting groups on compounds of Formula I can be removed according to standard synthetic methodologies (Theodora W. Greene and Peter G. M. Wuts, John Wiley and Sons, Inc., NY (1991)) and can be then subjected to further derivatization. Examples of further derivatization of compounds of I include, but are not limited to: when compounds of Formula I contain a primary or secondary amine, the amine may be reacted with aldehydes or ketones in the presence of a reducing agent such as sodium triacetoxyborohydride (see Abdel-Magid J.Org. Chem. 61, pp. 3849-3862, (1996)) to reductively alkylate; with acid chlorides or carboxylic acids and an amide bond forming reagent as described above to form amides; with sulfonyl chlorides to form sulfonamides; with isocyanates to form ureas; with aryl- or heteroaryl-halides in the presence of a palladium catalyst as described above (see Buchwald and Hartwig references above) to form aryl and heteroarylamines. In addition, when compounds of Formulae I contain an aryl halide or heteroaryl halide, these compounds may be subjected to metal-catalyzed reactions with boronic acids (for example, Suzuki or Stille couplings as described above), or, amines or alcohols (Buchwald- or Hartwig-type couplings, see Buchwald and Hartwig references above). When compounds of Formulae I contain a cyano group, this group may be hydrolyzed to amides or acids under acid or basic conditions. Basic amines may be oxidized to N-oxides and conversely N-oxides may be reduced to basic amines. When compounds of Formula I contain a sulfide, either acyclic or cyclic, the sulfide can be further oxidized to the corresponding sulfoxides or sulfones. Sulfoxides can be obtained by oxidation using an appropriate oxidant such as one equivalent of (*meta*-chloroperbenzoicacid) MCPBA or by treatment with NaIO₄ (see, for example, J. Regan, et al, J. Med. Chem., 46: 4676-86 (2003)) and sulfones can be obtained using two equivalents of MCPBA or by treatment with 4-methylmorpholine N-oxide and catalytic osmium tetroxide (see, for example, PCT application WO 01/47919).

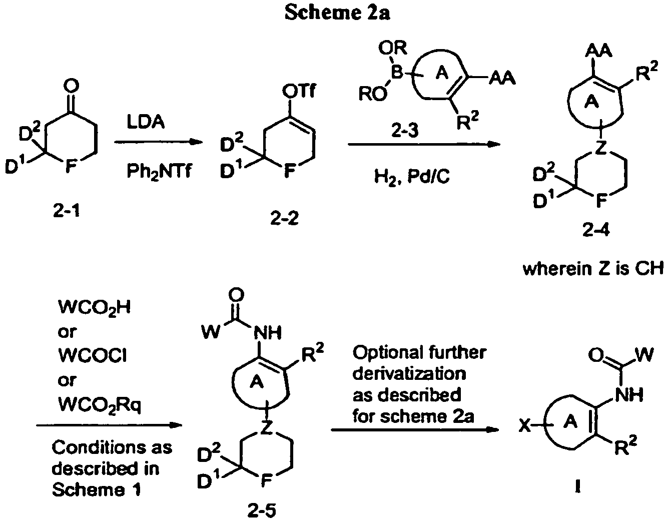

**Scheme 2a** illustrates a route to compounds of Formula I. F represents -NQₐQ_{b}R³-, - O-, S, SO, or SO₂, and AA represents ―NH₂ or -NO₂. D¹ and D² are shown for illustrative purposes only; it is recognized by those skilled in art that D⁵ D⁶ D⁷ D⁸ may also be present. Ketones of formula 2-1 can be converted to a vinyl triflate of formula 2-2 by treatment with a non-nucleophilic base such as LDA and then trapping of the resulting enolate with a triflating reagent such as trifluoromethanesulfonic anhydride or preferably N-phenyltrifluoromethanesulfonimide. Suzuki coupling of boronic acids or boronate esters of formula 2-3 to vinyl triflates of formula 2-2 can provide compounds of formula 2-4 where Z is C (synthesis, 993 (1991)).

For compounds of formula 2-4 treatment with Pd/C can reduce both the olefin (and the nitro if AA is NO₂) to give Z is CH, AA is NH₂. Compounds of formula 2-4 where F represents ―SO₂ can be prepared from compounds of formula 2-4 where AA is -NO₂ and F is a sulfide (F is -S-) by oxidation with MCPBA or other methods described in Scheme 1. The nitro group may then be reduced with Pd/C to reduce both the nitro and the olefin.

Compounds of formula 2-4 (AA is NH₂) are then converted to compounds of Formula 2-5 (which also represent compounds of Formulae I if no further modifications are required) as described in Scheme 1.

Compounds of formula 2-5 may be further modified to provide additional compounds of Formula 1. For example, in cases where F is ―NQₐQ_{b}R³-, QₐQ_{b} is a direct bond, and R₃ represents a BOC protecting group (CO₂tBu), the BOC group may be removed according to standard methodology such as trifluoroactic acid (TFA) in DCM (Greene and Wuts, *ibid*.) to provide a secondary amine that can then be further derivatized to provide compounds of Formula I. Further derivatization includes, but is not limited to: reactions with aldehydes or ketones in the presence of a reducing agent such as sodium triacetoxyborohydride to provide compounds of Formula II where F is - NCH₂R³ (A. F. Abdel-Magid, *ibid*.); with acid chlorides or with carboxylic acids and an amide bond forming reagent (as described in Scheme 1) to provide compounds of Formula II where F is -NCOR³; with sulfonyl chlorides (as described in Scheme 1) to provide compounds of Formula I where F is -NSO₂Rₐ; with isocyanates(as described in Scheme 1) to provide compounds of Formula II where F is -NCONRₐR_{b}; or subjected to metal-catalyzed substitution reactions as outlined in Scheme 1 to provide compounds of Formula I where F is -NR³. (S. L. Buchwald, et al, *ibid*.; J. H. Hartwig, *ibid.*) For the above example, Rₐ and R_{b} are independently hydrogen, alkyl, cycloalkyl, haloalkyl, aryl, aralkyl, heteroaryl and heteroaralkyl.

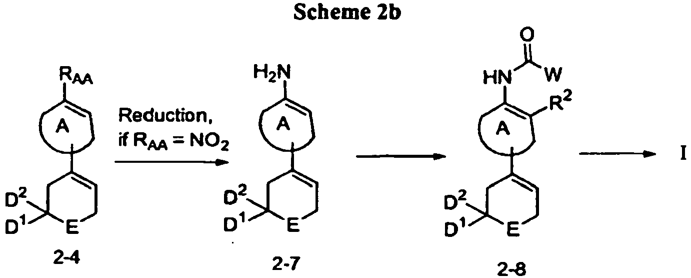

**Scheme 2b** illustrates a modification of Scheme 2a to synthesize partially unsaturated compounds of Formula I. E represents -NQₐQ_{b}R³-, -O- (D¹ and D² are H), -S- (D¹ and D² are H), -(D¹ and D² are H), or -SO₂- (D¹ and D² are H), and R_{AA} represents - NH₂ or -NO₂. Compounds of formula 2-4 are prepared as shown in Scheme 2. If R_{AA} is -NO₂, the nitro group must be reduced by a method that does not reduce olefins, such as iron and ammonium chloride. If R_{AA} of formula 2-4 is an amino group then no step is necessary and compounds of formula 2-4 are also compounds of formula 2-7. To prepared compounds of formula 2-7 where E is -SO₂- or -SO-, the oxidation of the sulfide must be performed on compound 2-4 where R_{AA} is -NO₂ as described above, followed by nitro reduction.

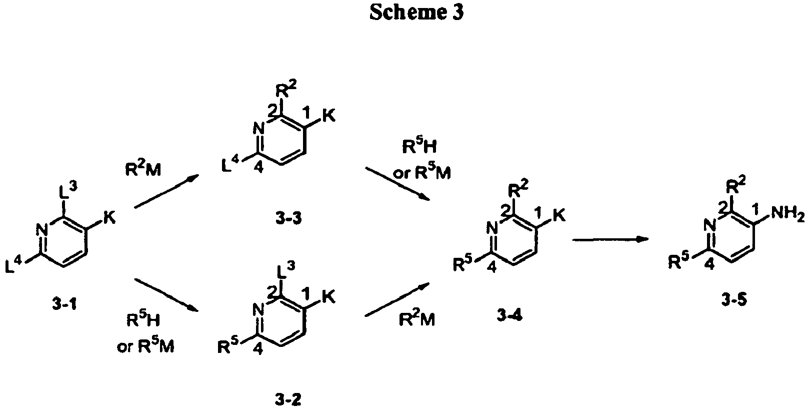

**Scheme 3** illustrates the preparation of intermediates for the synthesis of compounds of Formula I, where ring A is pyridyl, and R⁵ is the optional substitution on ring A or one of the heterocyclic substituents as defined in Formula I. K is NH₂ or other functional groups such as NO₂, COOH or COOR which can eventually be converted to amino group by known literature methods such as reductions for NO₂ (as discussed for Scheme 1) or Curtius rearrangement for COOH (for a review, see *Organic Reactions,* 3: 337 (1947)). L³ and L⁴ are halogens. (K is COOH can also be formed from K is COOR by simple base- or acid-catalyzed hydrolysis.) In general, the selectivity and order in introducing R² and R⁵ can be achieved by the relative reactivity of the halogens L³ and L⁴ chosen in compound (3-1), the intrinsic selectivity of the heterocycle and/or the reaction conditions employed. An example of using the relative reactivity of the halogens L³ and L⁴ in selectively introducing R² and R⁵ would include the situation where, in compounds of Formula 3-1 where L³ is a fluoro group and L⁴ is a bromo group, selective displacement of the fluoro group by a nucleophile can be achieved followed by substitution of the remaining bromo group by metal-catalyzed substitution chemistry (such as Suzuki or Stille cross-coupling reactions as further outlined below). Similarly in compounds of Formula 3-1 where one of L³ and L⁴ is an iodo group and the other is a bromo or chloro group, selective metal-catalyzed substitution chemistry (such as Suzuki or Stille cross-coupling reactions or Buchwald/Hartwig aminations as further discussed below) on the iodo group can be achieved followed by replacement of the remaining bromo or chloro group by another metal-catalyzed substitution reaction.

As illustrated in Scheme 3, leaving group L³ in Formula 3-1 can be first substituted to obtain compounds of Formula 3-3 or leaving group L⁴ can be first substituted to obtain compound of Formula 3-2. Compounds 3-2 or 3-3 can then be reacted to displace L³ or L⁴ to furnish the compound of Formula 3-4.

Thus, a direct nucleophilic displacement or metal-catalyzed amination of compound of Formula 3-1 with a secondary amine, ammonia or a protected amine such as *tert-*butyl carbamate (for review, see Modem Amination Methods: Ricci, A., Ed.; Wiley-VCH: Weinheim, 2000), can be used to introduce R⁵ in Formulae 3-2 or 3-3 where R⁵ is a primary or secondary amine, amino group (NH₂), and amine equivalent or a protected amino group. Metal-catalyzed coupling of compound 3-1 with boronic acids or boronates esters (Suzuki reaction, M is boronic acid group or boronate ester group) or with organotin compounds (Stille reaction, M is SnR₃, where R is alkyl and the other substituents as defined above, as described in Scheme I can provide compounds of Formulae 3-2 or 3-3.

Compound 3-2 can be further converted to compound 3-4 by a metal-catalyzed Suzuki or Stille coupling as described above. L⁴ in compound 3-3 also subsequently can be substituted with R⁵ to obtain compounds of Formula 3-4, again, by a direct nucleophilic substitution or metal-catalyzed reaction with a nucleophile or by the same metal-catalyzed cross-coupling reaction as described above. When R⁵ in the formulae (3-2, 3-3 or 3-4) is a protected amine and K not an amino group, it can be deprotected to unmask the amino functionality. This amino functionality can then be further derivatized as described in Scheme 1. When the K group in Formula 3-4 is not an amino group (such as functionality described above), it can be converted to an amino group according to known literature methods (see, for example Comprehensive Organic Transformations: Larock, R.S.; Wiley and Sons Inc., USA, 1999) and the resulting amine 3-5 can be employed in amide bond formation reactions as described in Scheme (1) to obtain the compounds in Formula I. When K in Formula 3-4 is an amino group it can be directly used in amide coupling as described above.

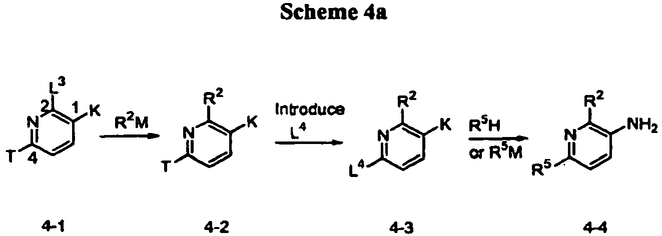

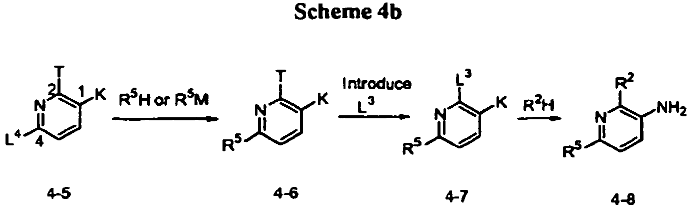

**Schemes 4a and 4b** illustrate the preparation of intermediates to be further modified according to Scheme 3 starting from a monohalo-substituted compound of Formulae 4-1 and 4-5 by introducing the second leaving group after the replacement of the first one has been completed. These can also be used for the synthesis of compounds of Formula I where ring A is a pyridine and R⁵ is either the optional substitution on Ring A or one of the heterocyclic substituents. As in Scheme 3, the remaining positions on the pyridine ring can be substituted as described in Formula 1. K is NH₂ or other functional groups such as NO₂, COOH or COOR which can eventually be converted to amino group by known literature methods such as reductions or Curtius rearrangement as described in Scheme 3. L³ and L⁴ are halogens. In these compounds, T is either H or is a functional group such as OH that can be converted to leaving groups L³ or L⁴ such as halogen, triflate or mesylate by known literature methods (see, for example, Nicolai, E., ct al., J. Heterocyclic Chemistry, 31, (73), (1994)). Displacement of L³ in compound of Formula 4-1 or L⁴ in Formula 4-5 by methods described in Scheme 3, can yield compounds of Formulae 4-2 and 4-6. At this point, the substituent T of compounds 4-2 or 4-6 can be converted to a leaving group L⁴ or L³ (preferably a halogen) by standard methods to provide compounds of Formulae 4-3 and 4-5. For example, when T is OH, the preferred reagents to effect this transformation are thionyl chloride, PCl₅, POCl₃ or PBr₃ (see, for examples, Kolder, den Hertog., Recl. Trav. Chim. Pays-Bas; 285, (1953), and Iddon, B, et. al., J. Chem. Soc. Perkin Trans. 1., 1370, (1980)). When T is H, it can be directly halogenated (preferably brominated) to provide compounds of Formulae 4-3 or 4-7 (see, for example, Canibano, V. et al., Synthesis, 14, 2175, (2001)). The preferred conditions for bromination are NBS in a suitable solvent such as DCM or acetonitrile. The compounds of Formulae 4-3 or 4-7 can be converted to compounds of Formulae 4-4 or 4-8 by introduction of the remaining groups R² or R⁵, respectively, by the methods described above and then on to compounds of Formula I, by the methods described in Scheme 3 for conversion of compounds of Formulae 3-4 and 3-5 to compounds of Formula I.

### Example 2

### 4-Cyano-1H-pyrrole-2-carboxylic acid

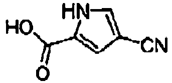

The title compound was prepared by the literature procedure (Loader and Anderson, Canadian J. Chem. 59: 2673 (1981)). ¹H-NMR (CDCl₃; 400 MHz): δ 12.70 (br s, 1H), 7.78 (s, 1H), 7.13 (s, 1H).

### Example 3

### 4-Cyano-1-(2-trimethylsilanyl-ethoxymethyl)-1H-imidazole-2-carboxylate potassium salt

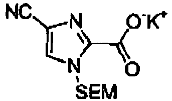

### a) 1-(2-Trimethylsilanyl-ethoxymethyl)-1H-imidazole-4-carbonitrile

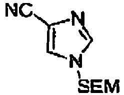

A flask charged with imidazole-4-carbonitrile (0.5 g, 5.2 mmol) (Synthesis, 677, 2003), 2-(trimethylsilyl)ethoxymethyl chloride (SEMCl) (0.95 mL, 5.3 mmol), K₂CO₃ (1.40 g, 10.4 mmol), and acetone (5 mL) was stirred for 10 h at RT. The mixture was diluted with EtOAc (20 mL) and washed with water (20 mL) and brine (20 mL) and the organic layer dried over MgSO₄. The crude product was eluted from a 20-g SPE cartridge (silica) with 30 % EtOAc/hexane to give 0.80 g (70 %) of the title compound as a colorless oil. Mass spectrum (CI (CH₄), m/z) Calcd. for C₁₀H₁₇N₃OSi, 224.1 (M+H), found 224.1.

### b) 2-Bromo-1-(2-trimethylsilanyl-ethoxymethyl)-1H-imidazole-4-carbonitrile

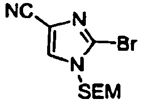

To a solution of 1-(2-trimethylsilanyl-ethoxymethyl)-1*H*-imidazole-4-carbonitrile (0.70 g, 3.1 mmol) (as prepared in the previous step) in CCl₄ (10 mL) was added NBS (0.61 g, 3.4 mmol) and AIBN (cat), and the mixture heated at 60 °C for 4 h. The reaction was diluted with EtOAc (30 mL) and washed with NaHCO₃ (2 x 30 mL) and brine (30 mL) and the organic layer was dried over Na₂SO₄ and then concentrated. The title compound was eluted from a 20-g SPE cartridge (silica) with 30 % EtOAc/hexane to give 0.73 g (77 %) of a yellow solid. Mass spectrum (CI (CH₄), m/z) Calcd. for C₁₀H₁₆BrN₃OSi, 302.0/304.0 (M+H), found 302.1/304.1.

### c) 4-Cyano-1-(2-trimethylsilanyl-ethoxymethyl)-1H-imidazole-2-carboxylic acid ethyl ester

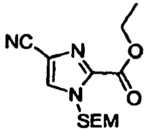

To a solution of 2-bromo-1-(2-trimethylsilanyl-ethoxymethyl)-1*H*-imidazole-4-carbonitrile (0.55 g, 1.8 mmol) (as prepared in the previous step) in THF (6 mL) at ― 40 °C was added drop wise a solution of 2M i-PrMgCl in THF (1 mL). The reaction was allowed to stir for 10 min at ―40 °C and then cooled to -78 °C, and ethyl cyanoformate (0.3 g, 3.0 mmol) was added. The reaction allowed to attain RT and stirred for 1 h. The reaction was quenched with satd aq NH₄Cl, diluted with EtOAc (20 mL) and washed with brine (2 x 20 mL), and the organic layer was dried over Na₂SO₄ and then concentrated. The title compound was eluted from a 20-g SPE cartridge (silica) with 30 % EtOAc/hexane to give 0.4 g (74 %) of a colorless oil. Mass spectrum (ESI, m/z): Calcd. for C₁₃H₂₁N₃O₃Si, 296.1 (M+H), found 296.1.

### d) 4-Cyano-1-(2-trimethylsilanyl-ethoxymethyl)-1H-imidazole-2-carboxylate potassium salt

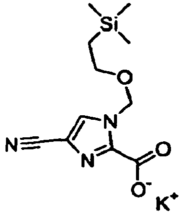

To a solution of 4-cyano-1-(2-trimethylsilanyl-ethoxymethyl)-1*H*-imidazole-2-carboxylic acid ethyl ester (0.4 g, 1.3 mmol) (as prepared in the previous step) in ethanol (3 mL) was added a solution of 6M KOH (0.2 mL) and the reaction was stirred for 10 min and then concentrated to give 0.40 g (100 %) of the title compound as a yellow solid. ¹H-NMR (400 MHz, CD₃OD) δ 7.98 (s, 1H), 5.92 (s, 2H), 3.62 (m, 2H), 0.94 (m, 2H), 0.00 (s, 9H). Mass spectrum (ESI-neg, m/z) Calcd. for C₁₁H₁₇N₃O₃Si, 266.1 (M-H), found 266.0.

### Example 13

### 4-Cyano-1H-pyrrole-2-carboxylic acid (2-cyclohex-1-enyl-4-piperidin-4-yl-phenyl)-amide trifluoroacetic acid salt

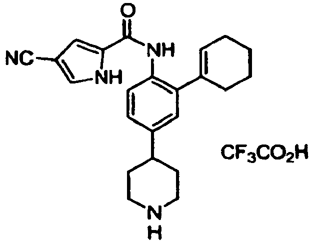

### a) 4-(4-Amino-phenyl)-3,6-dihydro-2H-pyridine-1-carboxylic acid tert-butyl ester

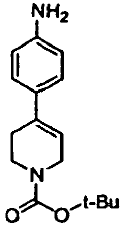

The title compound was prepared by Suzuki coupling of 4-(4,4,5,5-tetramethyl-[1,3,2]dioxaborolan-2-yl)-phenylamine with 4-trifluoromethanesulfonyloxy-3,6-dihydro-2*H*-pyridine-1-carboxylic acid tert-butyl ester (Synthesis, 993, (1991)) according to the procedure in Example 35, step (b). Mass spectrum (ESI, m/z): Calcd. for C₁₆H₂₂N₂O₂, 275.2 (M+H), found 275.1.

### b) 4-(4-Amino-phenyl)-piperidine-1-carboxylic acid tert-butyl ester

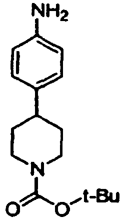

A solution of 4-(4-amino-phenyl)-3,6-dihydro-2*H*-pyridine-1-carboxylic acid tert-butyl ester (0.35 g, 1.2 mmol) (as prepared in the previous step) in methanol was hydrogenated over 10 % Pd/C at 20 psi for 1 h. The solution was filtered and concentrated to give 0.35 g (100 %) of the title compound as a yellow solid: Mass spectrum (ESI, m/z): Calcd. for C₁₆H₂₄N₂O₂, 277.2 (M+H), found 277.1.

### c) 4-(4-Amino-3-bromo-phenyl)-piperidine-1-carboxylic acid tert-butyl ester

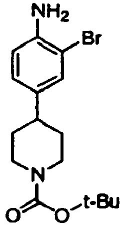

To a solution of 4-(4-amino-phenyl)-piperidine-1-carboxylic acid tert-butyl ester (0.20 g, 0.71 mmol) (as prepared in the previous step) in DCM (3 mL) was added N-bromosuccinimide (NBS) (0.13 g, 0.71 mmol), and the reaction stirred at RT for 10 h. The reaction was diluted with EtOAc (10 mL) and washed with NaHCO₃ (2 x 10 mL) and brine (10 mL). Concentration of the organic layer gave 0.26 g (100 %) of the title compound as a yellow foam. Mass spectrum (ESI, m/z): Calcd. for C₁₆H₂₃BrN₂O₂, 355.1 (M+H), found 355.1.

### d) 4-(4-Amino-3-cyclohex-1-enyl-phenyl)-piperidine-1-carboxylic acid tert-butyl ester

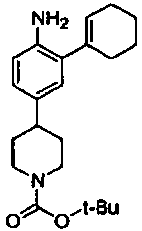

A flask was charged with 4-(4-amino-3-bromo-phenyl)-piperidine-1-carboxylic acid tert-butyl ester (0.13 g, 0.36 mmol) (as prepared in the previous step), cyclohex-1-enyl boronic acid (0.060 g, 0.48 mmol), Pd(PPh₃)₄ (0.04 g, 10 mol %), aqueous 2M Na₂CO₃ (1.5 mL), ethanol (1.5 mL), and toluene (3 mL), and heated at 80 °C for 3 h. The reaction was diluted EtOAc (10 mL), washed with NaHCO₃ (2 x 10 mL) and brine (10 mL), and the organic layer was dried over Na₂SO₄ and then concentrated. The title compound was eluted from a 20-g SPE cartridge (silica) with 30 % EtOAc/hexane to give 0.10 g (85 %) of the title compound as a yellow oil. Mass spectrum (ESI, m/z): Calcd. for C₂₂H₃₂N₂O₂, 357.2 (M+H), found 357.1.

### e) 4-Cyano-1H-pyrrole-2-carboxylic acid (2-cyclohex-1-enyl-4-piperidin-4-yl-phenyl)-amide trifluoroacetic acid salt

A flask was charged with 4-(4-amino-3-cyclohex-1-enyl-phenyl)-piperidine-1-carboxylic acid tert-butyl ester (0.050 g, 0.14 mmol) (as prepared in the previous step), 4-cyano-1H-pyrrole-2-carboxylic acid (0.019 g, 0.14 mmol)(as prepared in Example 2), EDCI (0.040 g, 0.21 mmol), HOBt (0.019 g, 0.14 mmol), DIEA (0.073 mL, 0.42 mmol), and DCM (0.5 mL) and stirred at 25 °C for 10 h. The reaction was loaded directly on a 10-g solid phase extraction (SPE) cartridge (silica) and the resulting intermediate was eluted with 30 % EtOAc/hexane. This compound was stirred at RT for 1 h in 50 % TFA/DCM (2 mL) and then concentrated and purified by RP-HPLC (C18), eluting with 30-50 % CH₃CN in 0.1 % TFA/H₂O over 12 min to give the title compound (0.052 g, 77 %). ¹H-NMR (400 MHz, CD₃OD): δ 7.59 (s, 1H), 7.50 (d, I H), 7.22 (d, 1H), 7.16 (m, 2H), 5.74 (m, 1H), 3.54. (m, 2H), 3.16 (m, 2H), 2.94 (m, 1H), 2.29 (m, 2H), 2.15 (m, 4H), 1.92 (m, 2H), 1.72 (m, 4H). Mass spectrum (ESI, m/z): Calcd. for C₂₃H₂₆N₄O, 375.2 (M+H), found 375.1.

### Example 14

### 4-Cyano-1H-imidazole-2-carboxylic acid (2-cyclohex-1-enyl-4-piperidin-4-yl-phenyl)-amide trifluoroacetic acid salt

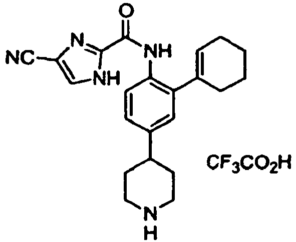

### a) 4-(4-{[4-Cyano-1-(2-trimethylsilanyl-ethoxymethyl)-1H-imidazole-2-carbonyl]-amino}-3-cyc/ohex-1-enyl-phenyl)-piperidine-1-carboxylic acid tert-butyl ester

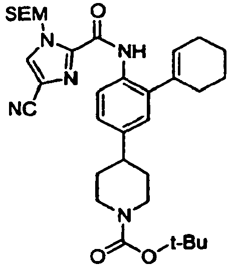

To a solution of 4-cyano-1-(2-trimethylsilanyl-ethoxymethyl)-1*H*-imidazole-2-carboxylate potassium salt (3.34 g, 10.9 mmol) (as prepared in Example 3, step (d)) in 20 mL DCM was added DIEA (3.8 mL, 21.8 mmol) and PyBroP (5.6 g, 12.0 mmol), and the reaction stirred at 25 °C for 15 min. A solution of 4-(4-amino-3-cyclohex-1-enyl-phenyl)-piperidine-1-carboxylic acid tert-butyl ester (3.9 g, 10.9 mmol) (as prepared in Example 13, stop (d)) in 10 mL DCM was added and the reaction stirred for 8 h at 25 °C. The reaction was diluted EtOAc (60 mL) and washed with NaHCO₃ (2 x 60 mL) and brine (100 mL) and the organic layer was dried over Na₂SO₄ and then concentrated. The title compound was purified by flash chomatography (silica gel, 2 % EtOAc/DCM to give 5.5 g (85 %) of the title compound as a yellow oil. Mass spectrum (ESI, m/z): Calcd. for C₂₃H₄₇N₅O₄Si, 606.2 (M+H), found 606.2.

### b) 4-Cyano-1H-imidazole-2-carboxylic acid (2-cyclohex-1-enyl-4-piperidin-4-yl-phenyl)-amide trifluoroacetic acid salt

To a solution of 4-(4-{[4-cyano-1-(2-trimethylsilanyl-ethoxymethyl)-1*H*-imidazole-2-carbonyl]-amino}-3-cyclohex-1-enyl-phenyl)-piperidine-1-carboxylic acid tert-butyl ester (1.5 g, 2.5 mmol) (as prepared in the previous step) in 10 mL of DCM and 0.3 mL EtOH was added 3 mL of TFA and the solution stirred for 3 h at 25 °C. The reaction was diluted with 5 mL of EtOH and then concentrated. The residue was crystallized from methanol and ethyl ether to give 0.85 g (70 %) of the title compound as a white solid. ¹H-NMR (400 MHz, CD₃OD) δ 8.18 (d, 1H), 8.04 (s, 1H), 7.22 (dd, 1H), 7.12 (d, 1H), 5.76 (m, 1H), 3.54. (m, 2H), 3.16 (m, 2H), 2.92 (m, 1H), 2.30 (m, 4H), 2.10 (m, 2H), 1.75 (m, 6H). Mass spectrum (ESI, m/z): Calcd. for C₂₂H₂₅N₅O, 376.2 (M+H), found 376.2.

**Reference Example 35**

### 4-Cyano-1H-imidazole-2-carboxylic acid [2-(1,1-dioxo-1,2,3,6-tetrahydro-1λ⁶-thiopyran-4-yl)-4-piperidin-4-yl-phenyl]-amide

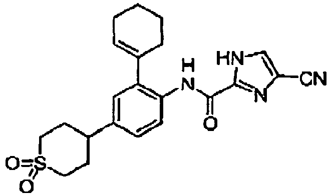

### a) Trifluoromethanesulfonic acid 3,6-dihydro-2H-thiopyran-4-yl ester

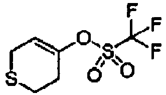

A solution of tetrahydro-thiopyran-4-one (1.00 g, 8.61 mmol) in 10 ml of THF was added to a solution of LDA (2.0 M, 4.52 ml, 9.04 mmol) in 20 ml of THF at ― 78 °C under Ar. The mixture was warmed to RT and stirred for 0.5 h, then cooled to - 78 °C again. A solution of *N*-phenyltrifluoromethanesulfonimide (3.42 g, 9.47 mmol) in 10 ml of THF was added. The resulting mixture was warmed to RT and stirred for 0.5 h under Ar. Treated with 200 ml of EtOAc, the mixture was washed with H₂O (3 x 50 mL), brine (50 mL) and dried (Na₂SO₄). Removal of the solvent under reduced pressure followed by flash chromatography of the residue on silica gel (hexane-3 % EtOAc/hexane) gave 810 mg (38 %) of the title compound as a colorless oil. ¹H-NMR (CDCl₃; 400 MHz): δ 6.01 (m, 1H), 3.30 (m, 2H), 2.86 (dd, 2H, J = 5.7, 5.7 Hz), 2.58-2.64 (m, 2H). Mass spectrum (ESI, m/z): Calcd. for C₆H₇F₃O₃S₂, 249.0 (M+H), found 249.3.

### b) 4-(4-Nitro-phenyl)-3,6-dihydro-2H-thiopyran

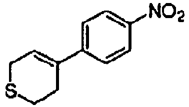

To a mixture of 4-nitrophenylboronic acid (418 mg, 2.50 mmol), trifluoromethanesulfonic acid 3,6-dihydro-2*H*-thiopyran-4-yl ester (as prepared in the previous step, 931 mg, 3.75 mmol), Pd(PPh₃)₄ (433 mg, 0.375 mmol) and lithium chloride (LiCl) (212 mg, 5.0 mmol) in 20 mL of 1,4-dioxane was added 2.0 M aq Na₂CO₃ solution (3.13 mL, 6.25 mmol). The resulting mixture was stirred at 80 °C for 2 h and then cooled to RT. Treated with 200 mL of EtOAc, the mixture was washed with H₂O (2 x 30 mL), brine (30 mL) and dried (Na₂SO₄). Removal of the solvent under reduced pressure followed by flash chromatography of the residue on silica gel (1-3 % EtOAc/hexane) gave 470 mg (85 %) of the title compound as a light brown oil. ¹H-NMR (CDCl₃; 400 MHz): δ 8.19 (d, 2H, J = 9.1 Hz), 7.48 (d, 2H, J = 9.1 Hz), 6.36 (m, 1H), 3.39 (m, 2H), 2.91 (t, 2H, J = 5.7 Hz), 2.72 (m, 2H). Mass spectrum (ESI, m/z): Calcd. for C₁₁H₁₁NO₂S, 222.1 (M+H), found 222.3.

### c) 4-(4-Nitro-phenyl)-3,6-dihydro-2H-thiopyran 1,1-dioxide

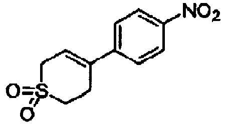

A solution of 3-chloroperoxybenzoic acid (1.04 g, 4.62 mmol, 77 %) in 15 mL of dichloromethane (DCM) was added slowly to a solution of 4-(4-nitro-phenyl)-3,6-dihydro-2*H*-thiopyran (as prepared in the previous step, 465 mg, 2.10 mmol) in 15 mL) of DCM at ― 78 °C under Ar. The mixture was stirred at ― 78 °C for 0.5 h, and then warmed to RT. Treated with 100 mL of EtOAc, the mixture was washed with 10 % Na₂SO₃ (2 x 15 mL), satd aq NaHCO₃ solution (20 mL), H₂O (20 mL), brine (20 mL) and dried (Na₂SO₄), Removal of the solvent under reduced pressure followed by flash chromatography of the residue on silica gel (2-5 % EtOAc/DCM) gave 518 mg (97 %) of the title compound as a white solid. ¹H-NMR (CDCl₃; 400 MHz): δ 8.23 (d, 2H, J = 9.0 Hz), 7.52 (d, 2H, J = 9.0 Hz), 6.04 (m, 1H), 3.86 (m, 2H), 3.26-3.31 (m, 2H), 3.18-3.23 (m, 2H).

### d) 4-(1,1-Dioxo-hexahydro-1λ⁶-thiopyran-4-yl)-phenylamine

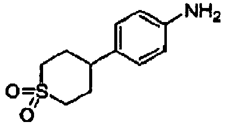

A mixture of 4-(4-nitro-phenyl)-3,6-dihydro-2*H*-thiopyran 1,1-dioxide (as prepared in the previous step, 502 mg, 1.98 mmol) and 10 % Pd/C (250 mg, 50 wt %) in 15 mL of MeOH was stirred at RT under H₂ (balloon pressure) for 2 h. The Pd catalyst was removed by filtration on Celite, and the filtrate was concentrated to give 314 mg (70 %) of the title compound as a slightly yellow solid. ¹H-NMR (CDCl₃; 400 MHz): δ 7.03 (d, 2H, J = 8.3 Hz), 6.67 (d, 2H, J = 8.3 Hz), 3.51-3.79 (br s, 2H), 3.11-3.17 (m, 4H), 2.70 (dddd, IH, J = 12.3, 12.3, 2.9, 2.9 Hz), 2.31-2.43 (m, 2H), 2.15-2.23 (m, 2H).

### e) 2-Bromo-4-(1,1-dioxo-hexahydro-1λ⁶-thiopyran-4-yl)-phenylamine

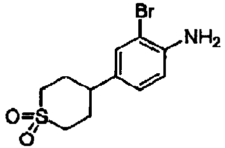

To a suspension of 4-(1,1-dioxo-hexahydro-1λ⁶-thiopyran-4-yl)-phenylamine (as prepared in the previous step, 174 mg, 0.77 mmol) in 20 mL) of 3:1 DCM/MeOH at 0 °C was added N-bromosuccinimide (NBS) (137 mg, 0.77 mmol) in 5 mL of DCM under Ar. The mixture was warmed to RT and stirred for 1 h under Ar. Treated with 100 mL of EtOAc, the mixture was washed with H₂O (2 x 20 mL), brine (20 mL) and dried (Na₂SO₄). Removal of the solvent under reduced pressure followed by flash chromatography of the residue on silica gel (2-3 % EtOAc/DCM) gave 155 mg (66 %) of the title compound as a white solid. ¹H-NMR (CDCl₃; 400 MHz): δ 7.28 (d, 1H, J = 2.0 Hz), 6.97 (dd, 1H, J = 8.3, 2.0 Hz), 6.73 (d, 1H, J = 8.3 Hz), 4.07 (br *s*, 2H), 3.09-3.14 (m, 4H), 2.66 (dddd, 1H, J = 12.1, 12.1, 3.3, 3.3 Hz), 2.26-2.39 (m, 2H), 2.12-2.21 (m, 2H). Mass spectrum (ESI, m/z): Calcd. for C₁₁H₁₄BrNO₂S, 304.0 (M+H), found 304.1.

### f) 2-Cyclohex-1-enyl-4-(1,1-dioxo-hexahydro-1λ⁶-thiopyran-4-yl)-phenylamine

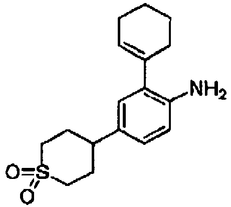

To a mixture of 2-bromo-4-(1,1-dioxo-hexahydro-1λ⁶-thiopyran-4-yl)-phenylamine (as prepared in the previous step, 150 mg, 0.493 mmol), cyclohexen-1-yl boronic acid (70 mg, 0.542 mmol) and Pd(PPh₃)₄ (57 mg, 0.0493 mmol) in 5 mL of 1,4-dioxane was added 2.0 M aq Na₂CO₃ solution (2.0 mL, 4.0 mmol). The resulting mixture was stirred at 80 °C for 8 h under Ar, and then cooled to RT. Treated with 50 mL of EtOAc, the mixture was washed with H₂O (3 x 15 mL), brine (20 mL) and dried (Na₂SO₄). Removal of the solvent under reduced pressure followed by flash chromatography of the residue on silica gel (2-5 % EtOAc/DCM) gave 130 mg (86 %) of the title compound as a brown solid. ¹H-NMR (CDCl₃; 400 MHz); δ 6.89 (dd, 1H, J = 8.4, 2.3 Hz), 6.84 (d, 1H, J = 2.3 Hz), 6.65 (d, 1H, J = 8.4 Hz), 5.74 (m, 1H), 3.74 (br s, 2H), 3.08-3.17 (m, 4H), 2.66 (dddd, 1H, J = 12.1, 12.1, 3.1, 3.1 Hz), 2.29-2.42 (m, 2H), 2.13-2.25 (m, 6H), 1.73-1.81 (m, 2H), 1.65-1.73 (m, 2H). Mass spectrum (ESI, m/z): Calcd. for C₁₇H₂₃NO₂S, 306.1 (M+H), found 306.1.

### g) 4-Cyano-1-(2-trimethylsilanyl-ethoxymethyl)-1H-imidazole-2-carboxylic acid [2-cyclohex-1-enyl-4-(1,1-dioxo-hexahydro-1λ⁶-thiopyran-4-yl)-phenyl]-amide

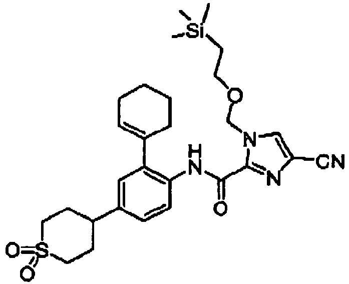

To a mixture of 2-cyclohex-1-enyl-4-(1,1-dioxo-hexahydro-1λ⁶-thiopyran-4-yl)-phenylamine (as prepared in the previous step, 122 mg, 0.50 mmol), potassium 4-cyano-1-(2-trimethylsilanyl-ethoxymethyl)-1H-imidazole-2-carboxylate (as prepared in Example 3, step (d), 134 mg, 0.44 mmol) and bromotri(pyrrolidino)phosphonium hexafluorophosphate (PyBroP) (205 mg, 0.44 mmol) in 5 mL of DMF was added DIEA (209 µL, 1.20 mmol). The resulting mixture was stirred at RT for 18 h under Ar, cooled to RT. Treated with 50 mL of EtOAc, the mixture was washed with H₂O (3 x 10 mL), brine (10 mL) and dried (Na₂SO₄). Removal of the solvent under reduced pressure followed by flash chromatography of the residue on silica gel (1-3 % EtOAc/DCM) gave 161 mg (73 %) of the title compound as a colorless oil. ¹H-NMR (CDCl₃; 400 MHz): δ 9.69 (s, 1H), 8.29 (d, 1H, J = 8.4 Hz), 7.78 (s, 1H), 7.14 (dd, 1H, J = 8.4, 2.2 Hz), 7.04 (d, 1H, J = 2.2 Hz), 5.95 (s, 2H), 5.83 (m, 1H), 3.66 (t, 2H, J = 8.2 Hz), 3.11-3.20 (m, 4H), 2.77 (dddd, 1H, J = 12.1, 12.1, 3.2, 3.2 Hz), 2.35-2.47 (m, 2H), 2.17-2.33 (m, 6H), 1.74-1.89 (m, 4H), 0.97 (t, 2H, J = 8.2 Hz), 0.00 (s, 9H). Mass spectrum (ESI, m/z): Calcd. for C₂₈H₃₈N₄O₉SSi, 555.2 (M+H), found 555.3.

### h) 4-Cyano-1H-imidazole-2-carboxylic acid [2-cyclohex-1-enyl-4-(1,1-dioxo-hexahydro-1λ⁶-thiopyran-4-yl)-phenyl]-amide

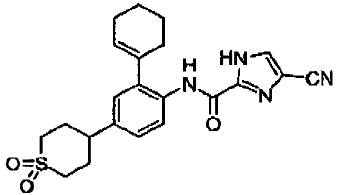

To a solution of 4-cyano-1-(2-trimethylsilanyl-ethoxymethyl)-1*H*-imidazole-2-carboxylic acid [2-cyclohex-1-enyl-4-(1,1-dioxo-hexahydro-1λ⁶-thiopyran-4-yl)-phenyl]-amide (as prepared in the previous step, 145 mg, 0.261 mmol) in 6 mL of DCM was added 0.20 mL of EtOH followed by 2 mL of TFA. The resulting solution was stirred at RT for 3 h. Removal of the solvent under reduced pressure followed by flash chromatography of the residue on silica gel (20-25 % EtOAc/DCM) gave 83 mg (90 %) of the title compound as a white solid. ¹H-NMR (CDCl₃; 400 MHz): δ 12.34 (s, 1H), 9.60 (s, 1H), 8.35 (d, 1H, J = 8.4 Hz), 7.75 (s, 1H), 7.30 (dd, 1H, J = 8.4, 2.2 Hz), 7.08 (d, 1H, J = 2.2 Hz), 5.86 (m, 1H), 3.11-3.23 (m, 4H), 2.80 (dddd, 1H, J = 12.2, 12.2, 2.8, 2.8 Hz), 2.40-2.57 (m, 2H), 2.17-2.35 (m, 6H), 1.74-1.91 (m, 4H). Mass spectrum (ESI, m/z): Calcd. for C₂₂H₂₄N₄O₃S, 425.2 (M+H), found 425.6.

### Example 38

### 4-Cyano-1H-imidazole-2-carboxylic acid {2-cyclohex-1-enyl-4-[1-(2-dimethylaminoacetyl)-piperidin-4-yl]-phenyl}-amide

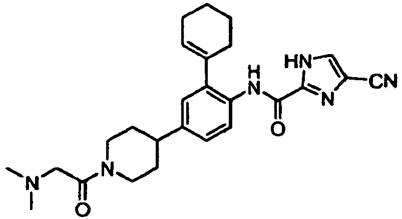

A mixture of 4-cyano-1*H*-imidazole-2-carboxylic acid (2-cyclohex-1-enyl-4-piperidin-4-yl-phenyl)-amide trifluoroacetic acid salt (as prepared in Example 14, step (b), 655 mg, 1.30 mmol) in DCM (15 mL) was cooled to 0 °C and DIEA (0.92 mL, 5.2 mmol) was added. Dimethylaminoacetyl chloride hydrochloride (211 mg, 1.3 mol) was then added portion wise over 10 min. The reaction mixture was stirred at 0 °C for 30 min and allowed to warm to RT and stirred for 2 h. Solvent was removed in vacuo and the resulting residue was partitioned between brine and DCM. The organic layer was separated, dried (Na₂SO₄) and concentrated. The residue obtained was purified on silica (5 % MeOH: DCM) to obtain 432 mg (70 %) of the title compound as a white solid. ¹H-NMR (CDCl₃; 400 MHz): δ 9.49 (s, 1H), 8.24 (d, 1H, J = 2.3 Hz), 7.70 (s, 1H), 7.12 (dd, 1H, J = 8.4, 2.1 Hz), 7.01 (s, 1H), 5.82 (m, 1H), 4.75 (d, 1H, J = 13.4 Hz), 4.13 (d, 1H, J = 13.4 Hz), 3.57 (d, 1H, J = 14.2 Hz), 3.18 (d, 1H, J = 14.2 Hz), 3.12 (td, 1H, J = 13.3, 2.4 Hz), 2.73 (dddd, 1H, J = 11.9, 11.9, 3.8, 3.8 Hz), 2.65 (ddd, 1H, J = 13.3, 13.3, 2.4 Hz), 2.40 (s, 6H), 2.18-2.32 (m, 4H), 1.60-1.98 (m, 8H). Mass spectrum (ESI, m/z): Calcd. for C₂₆H₃₂N₆O₂, 461.3 (M+H), found 461.2.

### BIOLOGICAL ACTIVITY

### In Vitro Assays

The following representative *in vitro* assays were performed in determining the C-KIT biological activity of the compounds of Formula I.

### c-Kit Fluorescence Polarization Kinase Assay

The compounds of the present invention are also specific inhibitors of c-Kit. Selection of compounds of Formula I for use as c-Kit inhibitors was performed in the following manner using an *in vitro* kinase assay to measure inhibition of the isolated kinase domain of the human c-kit receptor in a fluorescence polarization (FP) protocol. The c-kit assay utilized the fluorescein-labeled phosphopeptide and the anti-phosphotyrosine antibody included in the Panvera Phospho-Tyrosine Kinase Kit (Green) supplied by Invitrogen. When c-kit phosphorylated the poly Glu₄Tyr, the fluorescein-labeled phosphopeptide was displaced from the anti-phosphotyrosine antibody by the phosphorylated poly Glu₄Tyr, thus decreasing the FP value. The c-kit kinase reaction was incubated at room temperature for 45 minutes under the following conditions: 1nM c-kit (ProQinase, lot SP005), 100ug/mL poly Glu₄Tyr, 50uM ATP, 5mM MgCl_{2,} 1mM DTT, 0.01%Tween-20, 1% DMSO or compound in 100nM Hepes, pH 7.5. The kinase reaction was stopped with the addition of EDTA. The fluorescein-labeled phosphopeptide and the anti-phosphotyrosine antibody were added and incubated for 30 minutes at room temperature and fluorescence polarization was read. Data points were an average of triplicate samples. Inhibition and IC₅₀ data analysis were done with GraphPad Prism using a non-linear regression fit with a multiparamater, sigmoidal dose-response (variable slope) equation. The IC₅₀ for kinase inhibition represents the dose of a compound that resulted in a 50% inhibition of kinase activity compared to DMSO vehicle control.

### c-Kit BR-1 Assay

BR-1 cells are a dog mastocytoma line that expresses a constitutive-active mutant KIT (Ma Y., B.J. Longley, X. Wang, J.L. Blount, K. Langley, G.H. Caughey. Clustering of activating mutations in c-kit's juxtamembrane coding region in canine mast cell neoplasms. J Invest Dermatol 112: 165-170, 1999.). Proliferation of BR-1 cells can be inhibited by KIT inhibitors, and a good relationship exists between compound potency in KIT enzyme assays and inhibition of BR-1 proliferation. To assay inhibition of BR-1 cell proliferation, BR-1 cells are suspended (1 million cells/ml) in DMEM (Delbeccio's Modified Eagle's Media) containing 10%FCS, and 100 µl/well are plated into clear-bottomed 96 well culture plates (CoStar 3610) containing 50 µl of the same media supplemented with serial dilutions of test compounds.

Immediately following plating (time zero), 6 wells containing cells are treated with 100 µl ofPromega Cell TiterGlo reagent and incubated 10 min with shaking. The intensity of the Cell TiterGlo signal (1 sec/well) is determined using a luminometer. The remaining cells are cultured 72 hours (37 °C and 5% CO₂). Following the 72-hour growth interval, 100 µl of Promega Cell TiterGlo reagent is added to each well. The plates are incubated an additional 10 minutes with shaking and the intensity of the Cell TitcrGlo signal (1 sec/well) is determined using a luminometer. Cell proliferation is defined by the difference between the time zero and time 72 hour signals. IC₅₀ values of test compounds are calculated as the concentrations resulting in 50% inhibition of growth.

### BIOLOGICAL DATA

### Biological Data for C-KIT

The activity of compound number 38 is presented below. The activity is in µM and has the following uncertainties: C-KIT kinase: +10%.

| Compound Number | **Name** | C-KIT Fluorescence Polarization IC50 (µM) | C-KIT BR-1 Proliferation IC50 (µM) |
|---|---|---|---|
| **38** | 4-Cyano- 1H-imidazole-2-carboxylic acid {2-cyclohex-1-enyl-4-[1-(2-dimethylamino-acetyl)-piperidin-4-yl]-phenyl}- | 0.039 | 0.09 |

### COMPOUNDS FOR USE IN METHODS OF TREATMENT/PREVENTION

The present invention comprises compounds of the present invention for use in treating to disorders related to C-KIT kinase activity or expression in a subject, wherein said disorder is gastrointestinal stromal tumor or mastocytosis.

The kinase activity of C-KIT in a cell or a subject can be determined by procedures well known in the art, such as the C-KIT kinase assay described herein.

The term "subject" as used herein, refers to an animal, preferably a mammal, most preferably a human, who has been the object of treatment, observation or experiment.

In another example, the invention pertains to compounds for use in methods of treating in a subject gastrointestinal stromal tumor or mastocytosis comprising administering to the subject a therapeutically effective amount of a pharmaceutical composition comprising a compound of the present invention and a pharmaceutically acceptable carrier. Administration of said therapeutic agent can occur concurrently with the manifestation of symptoms characteristic of the disorder, such that said therapeutic agent serves as a therapy to compensate for the disorder.

The term "therapeutically effective amount" as used herein, refers to an amount of active compound or pharmaceutical agent that elicits the biological or medicinal response in a subject that is being sought by a researcher, veterinarian, medical doctor or other clinician, which includes alleviation of the symptoms of the disease or disorder being treated.

Methods for determining therapeutically and prophylactically effective doses for pharmaceutical compositions comprising a compound of the present invention are disclosed herein and known in the art.

As used herein, the term "composition" is intended to encompass a product comprising the specified ingredients in the specified amounts, as well as any product which results, directly or indirectly, from combinations of the specified ingredients in the specified amounts.

In a further embodiment to this aspect, the invention encompasses compounds for use in a combination therapy for treating gastrointestinal stromal tumor or mastocytosis. The combination therapy comprises administering to the subject a therapeutically effective amount of a compound of the present invention, and one or more other anti-cell proliferation therapy including chemotherapy, radiation therapy, gene therapy and immunotherapy.

In an embodiment of the present invention, a compound of the present invention may be administered in combination with chemotherapy. As used herein, chemotherapy refers to a therapy involving a chemotherapeutic agent. A variety of chemotherapeutic agents may be used in the combined treatment methods disclosed herein. Chemotherapeutic agents contemplated as exemplary, include, but are not limited to: platinum compounds (e.g.,cisplatin, carboplatin, oxaliplatin); taxane compounds (e.g., paclitaxcel, docetaxol); campotothecin compounds (irinotecan, topotecan); ; vinca alkaloids (e.g., vincristine, vinblastine, vinorelbine); anti-tumor nucleoside derivatives (e.g., 5-fluorouracil, leucovorin, gemcitabine, capecitabine) ; alkylating agents (e.g., cyclophosphamide, carmustine, lomustine, thiotepa); epipodophyllotoxins / podophyllotoxins (e.g. etoposide, teniposide); aromatase inhibitors (e.g., anastrozole, letrozole, exemestane); anti-estrogen compounds (e.g., tamoxifen, fulvestrant), antifolates (e.g., premetrexed disodium); hypomethylating agents (e.g., azacitidine); biologics (e.g., gemtuzamab, cetuximab, rituximab, pertuzumab, trastuzumab, bevacizumab, erlotinib); antibiotics/anthracyclines (e.g. idarubicin, actinomycin D, bleomycin, daunorubicin, doxorubicin, mitomycin C, dactinomycin, carminomycin, daunomycin); antimetabolites (e.g., aminopterin, clofarabine, cytosine arabinoside, methotrexate); tubulin-binding agents (e.g. combretastatin, colchicine, nocodazole); topoisomerase inhibitors (e.g., camptothecin). Further useful agents include verapamil, a calcium antagonist found to be useful in combination with antincoplastic agents to establish chemosensitivity in tumor cells resistant to accepted chemotherapeutic agents and to potentiate the efficacy of such compounds in drug-sensitive malignancies. See Simpson WG, The calcium channel blocker verapamil and cancer chemotherapy. Cell Calcium. 1985 Dec;6(6):449-67. Additionally, yet to emerge chemotherapeutic agents are contemplated as being useful in combination with a compound of the present invention.

In another embodiment of the present invention, the compounds of the present invention may be administered in combination with radiation therapy. As used herein, "radiation therapy" refers to a therapy that comprises exposing the subject in need thereof to radiation. Such therapy is known to those skilled in the art. The appropriate scheme of radiation therapy will be similar to those already employed in clinical therapies wherein the radiation therapy is used alone or in combination with other chemotherapeutics.

In another embodiment of the present invention, the compounds of the present invention may be administered in combination with a gene therapy. As used herein, "gene therapy" refers to a therapy targeting on particular genes involved in tumor development. Possible gene therapy strategies include the restoration of defective cancer-inhibitory genes, cell transduction or transfection with antisense DNA corresponding to genes coding for growth factors and their receptors, RNA-based strategies such as ribozymes, RNA decoys, antisense messenger RNAs and small interfering RNA (siRNA) molecules and the so-called'suicide genes'.

In other embodiments of this invention, the compounds of the present invention may be administered in combination with an immunotherapy. As used herein, "immunotherapy" refers to a therapy targeting particular protein involved in tumor development via antibodies specific to such protein. For example, monoclonal antibodies against vascular endothelial growth factor have been used in treating cancers.

Where a second pharmaceutical is used in addition to a compound of the present invention, the two pharmaceuticals may be administered simultaneously (e.g. in separate or unitary compositions) sequentially in either order, at approximately the same time, or on separate dosing schedules. In the latter case, the two compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular chemotherapeutic agent being administered in conjunction with the compound of the present invention, their route of administration, the particular tumor being treated and the particular host being treated.

As will be understood by those of ordinary skill in the art, the appropriate doses of chemotherapeutic agents will be generally similar to or less than those already employed in clinical therapies wherein the chemotherapeutics are administered alone or in combination with other chemotherapeutics.

The optimum method and order of administration and the dosage amounts and regime can be readily determined by those skilled in the art using conventional methods and in view of the information set out herein.

By way of example only, platinum compounds are advantageously administered in a dosage of 1 to 500 mg per square meter (mg/m²) of body surface area, for example 50 to 400 mg/m², particularly for cisplatin in a dosage of about 75 mg/m² and for carboplatin in about 300mg/m² per course of treatment. Cisplatin is not absorbed orally and must therefore be delivered via injection intravenously, subcutaneously, intratumorally or intraperitoneally.

By way of example only, taxane compounds are advantageously administered in a dosage of 50 to 400 mg per square meter (mg/m²) of body surface area, for example 75 to 250 mg/m², particularly for paclitaxel in a dosage of about 175 to 250 mg/m² and for docetaxel in about 75 to 150 mg/m² per course of treatment.

By way of example only, camptothecin compounds are advantageously administered in a dosage of 0.1 to 400 mg per square meter (mg/m²) of body surface area, for example 1 to 300 mg/m², particularly for irinotecan in a dosage of about 100 to 350 mg/m² and for topotecan in about 1 to 2 mg/m² per course of treatment.

By way of example only, vinca alkaloids may be advantageously administered in a dosage of 2 to 30 mg per square meter (mg/m²) of body surface area, particularly for vinblastine in a dosage of about 3 to 12 mg/m², for vincristine in a dosage of about 1 to 2 mg/m², and for vinorelbine in dosage of about 10 to 30 mg/m² per course of treatment.

By way of example only, anti-tumor nucleoside derivatives may be advantageously administered in a dosage of 200 to 2500 mg per square meter (mg/m²) of body surface area, for example 700 to 1500 mg/m². 5-fluorouracil (5-FU) is commonly used via intravenous administration with doses ranging from 200 to 500mg/m² (preferably from 3 to 15 mg/kg/day). Gemcitabine is advantageously administered in a dosage of about 800 to 1200 mg/m² and capecitabine is advantageously administered in about 1000 to 2500 mg/m² per course of treatment.

By way of example only, alkylating agents may be advantageously administered in a dosage of 100 to 500 mg per square meter (mg/m²) of body surface area, for example 120 to 200 mg/m², particularly for cyclophosphamide in a dosage of about 100 to 500 mg/m², for chlorambucil in a dosage of about 0.1 to 0.2 mg/kg of body weight, for carmustine in a dosage of about 150 to 200 mg/m², and for lomustine in a dosage of about 100 to 150 mg/m² per course of treatment.

By way of example only, podophyllotoxin derivatives may be advantageously administered in a dosage of 30 to 300 mg per square meter (mg/m2) of body surface area, for example 50 to 250 mg/m², particularly for etoposide in a dosage of about 35 to 100 mg/m² and for teniposide in about 50 to 250 mg/m² per course of treatment.

By way of example only, anthracycline derivatives may be advantageously administered in a dosage of 10 to 75 mg per square meter (mg/m²) of body surface area, for example 15 to 60 mg/m², particularly for doxorubicin in a dosage of about 40 to 75 mg/m², for daunorubicin in a dosage of about 25 to 45mg/m², and for idarubicin in a dosage of about 10 to 15 mg/m² per course of treatment.

By way of example only, anti-estrogen compounds may be advantageously administered in a dosage of about 1 to 100mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

By way of example only, biologics may be advantageously administered in a dosage of about 1 to 5 mg per square meter (mg/m²) of body surface area, or as known in the art, if different. For example, trastuzumab is advantageously administered in a dosage of 1 to 5 mg/m² particularly 2 to 4mg/m² per course of treatment.

Dosages may be administered, for example once, twice or more per course of treatment, which may be repeated for example every 7, 14, 21 or 28 days.

The compounds of the present invention can be administered to a subject systemically, for example, intravenously, orally, subcutaneously, intramuscular, intradermal, or parenterally. The compounds of the present invention can also be administered to a subject locally. Non-limiting examples of local delivery systems include the use of intraluminal medical devices that include intravascular drug delivery catheters, wires, pharmacological stents and endoluminal paving. A compound of the present invention can further be administered to a subject in combination with a targeting agent to achieve high local concentration of a compound at the target site. In addition, the compounds of the present invention may be formulated for fast-release or slow-release with the objective of maintaining the drugs or agents in contact with target tissues for a period ranging from hours to weeks.

The present invention also provides a pharmaceutical composition comprising a compound of the present invention in association with a pharmaceutically acceptable carrier. The pharmaceutical composition may contain between about 0.1 mg and 1000 mg, preferably about 100 to 500 mg, of the compound, and may be constituted into any form suitable for the mode of administration selected.

The phrase "pharmaceutically acceptable" refers to molecular entities and compositions that do not produce an adverse, allergic or other untoward reaction when administered to an animal, or a human, as appropriate. Veterinary uses are equally included within the invention and "pharmaceutically acceptable" formulations include formulations for both clinical and/or veterinary use.

Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

The pharmaceutical compositions of the present invention also include a pharmaceutical composition for slow release of the compounds of the present invention. The composition includes a slow release carrier (typically, a polymeric carrier) and a compound of the present invention.

Slow release biodegradable carriers are well known in the art. These are materials that may form particles that capture therein an active compound(s) and slowly degrade/dissolve under a suitable environment (e.g., aqueous, acidic, basic, etc) and thereby degrade/dissolve in body fluids and release the active compound(s) therein. The particles are preferably nanoparticles (i.e., in the range of about 1 to 500 nm in diameter, preferably about 50-200 nm in diameter, and most preferably about 100 nm in diameter).

Methods to prepare the pharmaceutical compositions of the invention are disclosed. A compound of the present invention, as the active ingredient, is intimately admixed with a pharmaceutical carrier according to conventional pharmaceutical compounding techniques, which carrier may take a wide variety of forms depending on the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, though other ingredients, for example, for purposes such as aiding solubility or for preservation, may be included. Injectable suspensions may also be prepared, in which case appropriate liquid carriers, suspending agents and the like may be employed. In preparation for slow release, a slow release carrier, typically a polymeric carrier, and a compound of the present invention are first dissolved or dispersed in an organic solvent. The obtained organic solution is then added into an aqueous solution to obtain an oil-in-water-type emulsion. Preferably, the aqueous solution includes surface-active agent(s). Subsequently, the organic solvent is evaporated from the oil-in-water-type emulsion to obtain a colloidal suspension of particles containing the slow release carrier and the compound of the present invention.

The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, from about 0.01 mg to 200 mg/kg of body weight per day. Preferably, the range is from about 0.03 to about 100 mg/kg of body weight per day, most preferably, from about 0.05 to about 10 mg/kg of body weight per day. The compound may be administered on a regimen of 1 to 5 times per day. The dosages, however, may be varied depending upon the requirement of the patients, the severity of the condition being treated and the compound(s) being employed. The use of either daily administration or post-periodic dosing may be employed.

Preferably these compositions are in unit dosage forms such as tablets, pills, capsules, powders, granules, sterile parenteral solutions or suspensions, metered aerosol or liquid sprays, drops, ampoules, auto-injector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of the active ingredient of the present invention. The tablets or pills of the composition of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, acetyl alcohol and cellulose acetate.

The liquid forms in which a compound of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin. The liquid forms in suitably flavored suspending or dispersing agents may also include the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Advantageously, the compounds of the present invention may be administered in a single daily dose, or the total daily dosage may be administered in divided doses of two, three or four times daily. Furthermore, compounds of the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The daily dosage of the products of the present invention may be varied over a wide range from 1 to 5000 mg per adult human per day. For oral administration, the compositions are preferably provided in the form of tablets containing, 0.01,0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated. An effective amount of a compound of the present invention is ordinarily supplied at a dosage level of from about 0.01 mg/kg to about 200 mg/kg of body weight per day. Particularly, the range is from about 0.03 to about 15 mg/kg of body weight per day, and more particularly, from about 0.05 to about 10 mg/kg of body weight per day. The compounds of the present invention may be administered on a regimen up to four or more times per day, preferably of I to 2 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

The compounds of the present invention can also be administered in the form of liposome delivery systems, such as small unilamellar vesicles, large unilamellar vesicles, and multilamellar vesicles. Liposomes can be formed from a variety of lipids, including but not limited to amphipathic lipids such as phosphatidylcholines, sphingomyelins, phosphatidylethanolamines, phophatidylcholines, cardiolipins, phosphatidylserines, phosphatidylglycerols, phosphatidic acids, phosphatidylinositols, diacyl trimethylammonium propanes, diacyl dimethylammonium propanes, and stearylamine, neutral lipids such as triglycerides, and combinations thereof. They may either contain cholesterol or may be cholesterol-free.

Another alternative method for administering the compounds of the invention may be by conjugating a compound to a targeting agent which directs the conjugate to its intended site of action, i.e., to vascular endothelial cells, or to tumor cells. Both antibody and non-antibody targeting agents may be used. Because of the specific interaction between the targeting agent and its corresponding binding partner, the compound of the present invention can be administered with high local concentrations at or near a target site and thus treats the disorder at the target site more effectively.

The antibody targeting agents include antibodies or antigen-binding fragments thereof, that bind to a targetable or accessible component of a tumor cell, tumor vasculature, or tumor stroma. The "targetable or accessible component" of a tumor cell, tumor vasculature or tumor stroma, is preferably a surface-expressed, surface-accessible or surface-localized component. The antibody targeting agents also include antibodies or antigen-binding fragments thereof, that bind to an intracellular component that is released from a necrotic tumor cell. Preferably such antibodies are monoclonal antibodies, or antigen-binding fragments thereof, that bind to insoluble intracellular antigen(s) present in cells that may be induced to be permeable, or in cell ghosts of substantially all neoplastic and normal cells, but are not present or accessible on the exterior of normal living cells of a mammal.

As used herein, the term "antibody" is intended to refer broadly to any immunologic binding agent such as IgG, IgM, IgA, IgE, F(ab')2, a univalent fragment such as Fab', Fab, Dab, as well as engineered antibodies such as recombinant antibodies, humanized antibodies, bispecific antibodies, and the like. The antibody can be either the polyclonal or the monoclonal, although the monoclonal is preferred. There is a very broad array of antibodies known in the art that have immunological specificity for the cell surface of virtually any solid tumor type (see, Summary Table on monoclonal antibodies for solid tumors in US Patent No. 5,855,866 to Thorpe et al). Methods are known to those skilled in the art to produce and isolate antibodies against tumor (see, US Patent No.5,855,866 to Thorpe et al., and US Patent No.6,34,2219 to Thorpe et al.).

Techniques for conjugating therapeutic moiety to antibodies are well known. (See, e.g., Amon ct al., "Monoclonal Antibodies For Immunotargeting Of Drugs In Cancer Therapy", in Monoclonal Antibodies And Cancer Therapy, Reisfeld et al. (eds.), pp. 243- 56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985)). Similar techniques can also be applied to attach compounds of the invention to non-antibody targeting agents. Those skilled in the art will know, or be able to determine, methods of forming conjugates with non-antibody targeting agents, such as small molecules, oligopeptides, polysaccharides, or other polyanionic compounds.

Although any linking moiety that is reasonably stable in blood, can be used to link the compounds of the present invention to the targeting agent, biologically-releasable bonds and/or selectively cleavable spacers or linkers are preferred. "Biologically-releasable bonds" and "selectively cleavable spacers or linkers" still have reasonable stability in the circulation, but are releasable, cleavable or hydrolyzable only or preferentially under certain conditions, i.e., within a certain environment, or in contact with a particular agent. Such bonds include, for example, disulfide and trisulfide bonds and acid-labile bonds, as described in U.S. Pat. Nos. 5, 474,765 and 5,762,918 and enzyme-sensitive bonds, including peptide bonds, esters, amides, phosphodiesters and glycosides as described in U.S. Pat. Nos. 5,474,765 and 5,762,918. Such selective-release design features facilitate sustained release of the compounds from the conjugates at the intended target site.

The present invention further provides compounds for use in a method of treating gastrointestinal stromal tumor or mastocytosis comprising administering to a subject a therapeutically effective amount of a compound of the present invention conjugated to a targeting agent.

When proteins such as antibodies or growth factors, or polysaccharides are used as targeting agents, they are preferably administered in the form of injectable compositions. The injectable antibody solution will be administered into a vein, artery or into the spinal fluid over the course of from 2 minutes to about 45 minutes, preferably from 10 to 20 minutes. In certain cases, intradermal and intracavitary administration are advantageous for tumors restricted to areas close to particular regions of the skin and/or to particular body cavities. In addition, intrathecal administrations may be used for tumors located in the brain.

A therapeutically effective dose of a compound of the present invention conjugated to a targeting agent depends on the individual, the disease type, the disease state, the method of administration and other clinical variables. The effective dosages are readily determinable using data from animal models, including those presented herein.

## Claims

1. A compound of formula 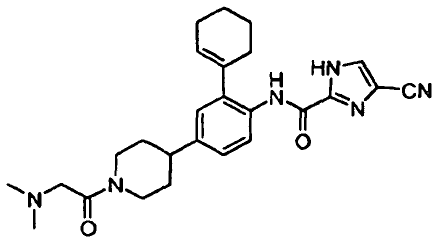 or a solvate, hydrate, tautomer or pharmaceutically acceptable salt thereof for use in a method of treating gastrointestinal stromal tumor or mastocytosis.

2. A compound of formula 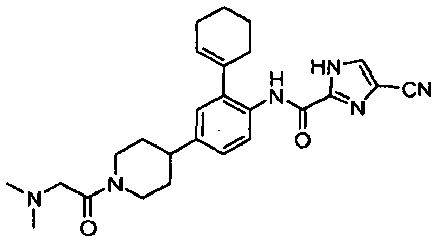 or a solvate, hydrate, tautomer or pharmaceutically acceptable salt thereof, and a pharmaceutically acceptable carrier for use in a method of treating gastrointestinal stromal tumor or mastocytosis.

3. A compound of formula 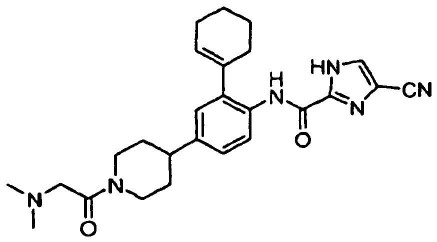 or a solvate, hydrate, tautomer or pharmaceutically acceptable salt thereof, conjugated to a targeting agent for use in a method of treating gastrointestinal stromal tumor or mastocytosis.

4. A compound of any of claims 1-3, wherein said method further comprises administration of a chemotherapeutic agent.

5. A compound of any of claims 1-3, wherein said method further comprises administration of radiation therapy.

## Patentansprüche

1. Verbindung der Formel 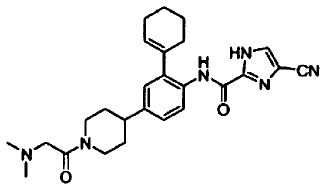 oder ein Solvat, Hydrat, Tautomer oder pharmazeutisch unbedenkliches Salz davon zur Verwendung in einem Verfahren zur Behandlung von gastrointestinalem Stromatumor oder Mastocytose.

2. Verbindung der Formel 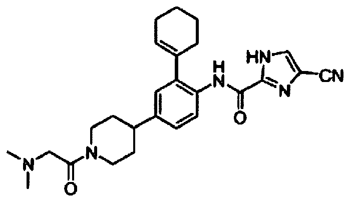 oder ein Solvat, Hydrat, Tautomer oder pharmazeutisch unbedenkliches Salz davon sowie ein pharmazeutisch unbedeklicher Trägerstoff zur Verwendung in einem Verfahren zur Behandlung von gastrointestinalem Stromatumor oder Mastocytose.

3. Verbindung der Formel 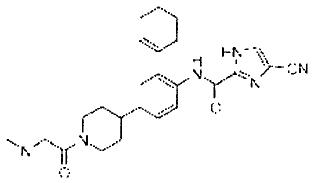 oder ein Solvat, Hydrat, Tautomer oder pharmazeutisch unbedenkliches Salz davon, konjugiert mit einem Zielmittel zur Verwendung in einem Verfahren zur Behandlung von gastrointestinalem Stromatumor oder Mastocytose.

4. Verbindung nach einem der Ansprüche 1-3, wobei man in dem Verfahren ferner ein Chemotherapeutikum verabreicht.

5. Verbindung nach einem der Ansprüche 1-3, wobei man in dem Verfahren ferner eine Strahlentherapie verabreicht.

## Revendications

1. Composé de formule 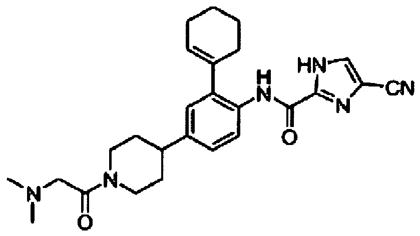 ou un solvat, hydrate, tautomère ou sel pharmaceutiquement acceptable de celui-ci, pour une utilisation dans une méthode de traitement d'une tumeur stromale gastro-intestinale ou d'une mastocytose.

2. Composé de formule 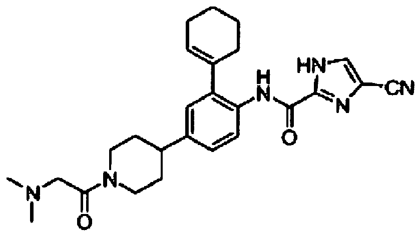 ou un solvat, hydrate, tautomère ou sel pharmaceutiquement acceptable de celui-ci, et un véhicule pharmaceutiquement acceptable, pour une utilisation dans une méthode de traitement d'une tumeur stromale gastro-intestinale ou d'une mastocytose.

3. Composé de formule 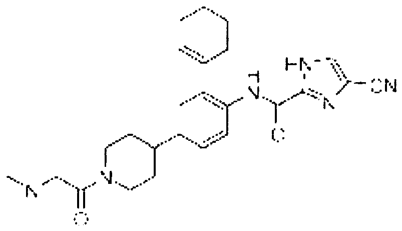 ou un solvat, hydrate, tautomère ou sel pharmaceutiquement acceptable de celui-ci, conjugué à un agent de vectorisation, pour une utilisation dans une méthode de traitement d'une tumeur stromale gastro-intestinale ou d'une mastocytose.

4. Composé selon l'une quelconque des revendications 1-3, **caractérisé en ce que** ladite méthode comprend en outre l'administration d'un agent chimiothérapeutique.

5. Composé selon l'une quelconque des revendications 1-3, **caractérisé en ce que** ladite méthode comprend en outre l'administration d'une radiothérapie.
